(19) Europäisches Patentamt / European Patent Office / Office européen des brevets

(11) **EP 3 474 984 B1**

(12) **FASCICULE DE BREVET EUROPEEN**

(45) Date de publication et mention
de la délivrance du brevet:
**11.03.2026 Bulletin 2026/11**

(21) Numéro de dépôt: **17732476.1**

(22) Date de dépôt: **28.06.2017**

(51) Classification Internationale des Brevets (IPC):
**B01J 20/18** (2006.01)    **B01J 20/28** (2006.01)
**B01J 20/30** (2006.01)    **C07C 7/13** (2006.01)

(52) Classification Coopérative des Brevets (CPC):
(C-Sets disponibles)
**B01J 20/186; B01J 20/28004; B01J 20/28011;
B01J 20/2803; B01J 20/28054; B01J 20/2808;
B01J 20/28092; B01J 20/3028; B01J 20/3071;
B01J 20/3085; C07C 7/13;** Y02P 20/52     (Cont.)

(86) Numéro de dépôt international:
**PCT/EP2017/066054**

(87) Numéro de publication internationale:
**WO 2018/002174 (04.01.2018 Gazette 2018/01)**

(54) **ADSORBANT ZÉOLITHIQUE SOUS FORME D'AGGLOMÉRÉS À FAIBLE TORTUOSITÉ**

ZEOLITHADSORBENS IN FORM VON AGGLOMERATEN MIT GERINGER TORTUOSITÄT

ZEOLITE ADSORBENT IN THE FORM OF LOW-TORTUOSITY AGGLOMERATES

(84) Etats contractants désignés:
**AL AT BE BG CH CY CZ DE DK EE ES FI FR GB
GR HR HU IE IS IT LI LT LU LV MC MK MT NL NO
PL PT RO RS SE SI SK SM TR**

(30) Priorité: **28.06.2016 FR 1656031**

(43) Date de publication de la demande:
**01.05.2019 Bulletin 2019/18**

(73) Titulaires:
• **IFP Energies nouvelles**
  **92852 Rueil-Malmaison (FR)**
• **Arkema France**
  **92800 Puteaux (FR)**

(72) Inventeurs:
• **LAROCHE, Catherine**
  **92852 RUEIL-MALMAISON CEDEX (FR)**
• **LEFLAIVE, Philibert**
  **69780 Mions (FR)**
• **BOUVIER, Ludivine**
  **64300 Orthez (FR)**
• **LUTZ, Cécile**
  **64290 Gan (FR)**

(74) Mandataire: **Bandpay & Greuter**
**11 rue Christophe Colomb**
**75008 Paris (FR)**

(56) Documents cités:
**WO-A1-2016/075280     FR-A1- 3 004 966**

• **NURADEEN LABARAN TANKO: "The effect of
Porosity on Tortuosity", INTERNATIONAL
JOURNAL OF SCIENTIFIC & ENGINEERING
RESEARCH, 1 May 2018 (2018-05-01), pages
2163 - 2169, XP055682752, Retrieved from the
Internet <URL:https://www.ijser.org/
researchpaper/
The-effect-of-Porosity-on-Tortuosity.pdf>
[retrieved on 20200403]**
• **DR PHYU ET AL: "Preparation of Synthetic
Zeolites From Myanmar Clay Mineral",
INTERNATIONAL NUCLEAR INFORMATION
SYSTEM (INIS) | IAEA, 1 April 2004 (2004-04-01),
pages 1 - 29, XP055349257, Retrieved from the
Internet <URL:http://www.iaea.org/inis/
collection/NCLCollectionStore/_Public/39/080/
39080137.pdf?r=1> [retrieved on 20170224]**

(52) Classification Coopérative des Brevets (CPC):
(Cont.)

C-Sets
**C07C 7/13, C07C 15/08**

**Description**

**Domaine de l'invention**

La présente invention concerne le domaine des adsorbants à structure zéolithique et plus particulièrement le domaine des adsorbants à structure zéolithique sous forme d'agglomérés. La présente invention concerne également la préparation desdits agglomérés à structure zéolithique, ainsi que leurs utilisations pour la séparation de mélanges gazeux ou liquides.

**Art antérieur**

La synthèse de zéolithes conduit à des cristaux, généralement sous forme de poudre, dont l'emploi à l'échelle industrielle est particulièrement malaisé. En effet, on sait aujourd'hui synthétiser des cristaux de zéolithe de taille variant de quelques nanomètres à quelques micromètres, tailles requises pour conférer aux zéolithes des capacités optimales d'adsorption. Les inconvénients liés à ces cristaux de petites tailles sont cependant nombreux, inconvénients parmi lesquels on peut citer la difficulté de manutention de poudre pulvérulente et les pertes de charge importantes lors de leur utilisation.

Afin de pallier ces inconvénients, il a donc été proposé d'utiliser des formes agglomérées de ces cristaux, par exemple sous forme de filés, de billes et autres formes agglomérées. La fabrication de tels agglomérés à partir de cristaux de zéolithe(s) sous forme de poudre est bien connue aujourd'hui, et la littérature scientifique et la littérature brevet fournissent de nombreux exemples de préparation d'agglomérés zéolithiques, notamment par extrusion, pastillage, et autres techniques d'agglomération connues de l'homme du métier.

Ces agglomérés sont habituellement de tailles de l'ordre de quelques dizaines de micromètres, voire de quelques centaines de micromètres, voire de quelques millimètres, et ne présentent pas les inconvénients inhérents aux matières pulvérulentes que sont les cristaux de zéolithes précédemment définis.

Ces agglomérés, qu'ils soient sous forme de plaquettes, de billes, d'extrudés, et autres, sont en général constitués de cristaux de zéolithe(s), qui constituent l'élément actif (au sens de l'adsorption) et d'un liant d'agglomération.

Ce liant d'agglomération est destiné à assurer la cohésion des cristaux entre eux dans la structure agglomérée, mais aussi doit permettre d'assurer une résistance mécanique suffisante auxdits agglomérés afin d'éviter, ou tout au moins de minimiser le plus possible, les risques de fractures, brisures ou cassures qui pourraient survenir lors de leurs utilisations industrielles pendant lesquelles les agglomérés sont soumis à de nombreuses contraintes, telles que vibrations, variations fortes et/ou fréquentes de pression, mouvements et autres. Il est donc très important que les agglomérés zéolithiques soumis à ces diverses contraintes restent cohésifs et ne puissent pas générer de fines particules pulvérulentes conduisant aux inconvénients précités. Il est donc très important de pouvoir disposer d'agglomérés zéolithiques présentant des propriétés améliorées, comme notamment une stabilité dans le temps améliorée, c'est-à-dire dont les performances de séparation dans le temps ne sont pas détériorées.

Cependant, les propriétés d'adsorption de ces agglomérés sont évidemment réduites par rapport à la poudre de cristaux, en raison de la présence de liant d'agglomération inerte vis-à-vis de l'adsorption.

Divers moyens ont déjà été proposés pour pallier cet inconvénient du liant d'agglomération d'être inerte quant aux performances d'adsorption, parmi lesquels, la transformation de la totalité ou d'au moins une partie du liant d'agglomération en zéolithe active du point de vue de l'adsorption. Cette opération est bien maintenant connue de l'homme du métier, par exemple sous la dénomination de « zéolithisation ». Pour effectuer facilement cette opération, on utilise des liants zéolithisables, le plus souvent des argiles appartenant à la famille de la kaolinite, et de préférence préalablement calcinés à des températures généralement comprises entre 500°C et 700°C, c'est-à-dire visant à cuire l'argile (« calcinate » ou « fire » en langue anglaise).

Les facteurs importants qui influencent les performances d'un procédé de séparation par adsorption englobent notamment la sélectivité d'adsorption, la capacité d'adsorption et la cinétique de transfert de matière au sein de l'adsorbant qui contrôle les vitesses d'adsorption et de désorption des différents composés. L'adsorbant doit donc présenter de bonnes propriétés de transfert de matière afin de garantir un nombre de plateaux théoriques suffisants pour réaliser une séparation efficace des espèces en mélange, comme l'indique Ruthven dans l'ouvrage intitulé « Principles of Adsorption and Adsorption Processes » (« Principes de l'Adsorption et des Procédés d'Adsorption »), John Wiley & Sons, (1984), pages 326 et 407. Pour estimer l'amélioration de la cinétique de transfert, il est possible d'utiliser la théorie des plateaux décrite dans l'ouvrage cité, pages 248-250. Cette approche est basée sur la représentation d'une colonne par un nombre fini de réacteurs hypothétiques idéalement agités (étages théoriques). La hauteur équivalente de plateaux théoriques est une mesure directe de la dispersion axiale et de la résistance au transfert de matière du système.

Ruthven précise (ibid., page 124) qu'en général, le transport des molécules au cœur du réseau poreux de l'adsorbant se fait non pas par écoulement, mais par diffusion. Le mécanisme de diffusion dans le réseau poreux dépend notamment de la taille des pores, et par conséquent, au sein de l'adsorbant constitué de cristaux de zéolithe agglomérés, deux régimes de diffusion coexistent : la diffusion intra-cristalline dans les micropores de la zéolithe et la diffusion intra-particulaire dans les macropores et mésopores de l'espace inter-cristallin. Le temps de transfert global au sein d'un adsorbant zéolithique sous forme d'aggloméré sera alors l'addition du temps de transfert par diffusion intra-particulaire à travers le réseau macro- et méso-poreux et du temps de transfert par diffusion intra-cristalline. Or le temps caractéristique de transfert par diffusion au sein d'une particule de géométrie sphérique s'écrit de façon générale comme le carré du rayon de la particule divisé par la diffusivité des molécules transportées au sein de cette particule. Par conséquent, dans le but d'améliorer le transfert de matière au sein des adsorbants zéolithiques sous forme d'aggloméré, on cherchera avant toute chose à réduire la taille des cristaux de zéolithe et/ou la taille des agglomérés.

En pratique, on cherchera à réduire la taille des agglomérés, mais seulement jusqu'à un certain point, car ce paramètre détermine la perte de charge et l'uniformité du remplissage au sein de l'unité industrielle lors de l'utilisation de l'adsorbant dans l'application industrielle.

De même la réduction de la taille des cristaux permet d'améliorer le transfert microporeux, mais risquerait d'avoir une incidence sur la diffusivité intra-particulaire dans les macropores. Par conséquent, il n'est pas souhaitable de réduire trop fortement la taille des cristaux, au risque de réduire la diffusivité intra-particulaire à un niveau non acceptable. D'autres voies d'amélioration du transfert restent donc à investiguer. Le document WO 2016/075280 concerne un adsorbant comprenant une phase zéolithique comprenant au moins une zéolithe de structure FAU de type X et une phase non zéolithique. Cet adsorbant présente une surface externe inférieure ou égale à 30 $m^2$/g et une distribution de diamètre de pores de 100 à 250 nm. Le document FR 3004966 concerne un adsorbant zéolitique à base de cristaux agglomérés de zéolithe EMT comprenant du baryum et/ou du potassium.

Lorsque des molécules diffusent à travers un matériau poreux, leur diffusivité effective à travers le réseau poreux $D_p$ est réduite par rapport à leur diffusivité $D_0$ lorsqu'elles diffusent dans un volume libre. Le facteur de réduction est égal au rapport de la porosité $\varepsilon_p$ avec le facteur de tortuosité $\tau$ du matériau poreux, selon l'Équation 1 (ouvrage de Kärger, Ruthven et Theodorou, « Diffusion in Nanoporous Materials », Volume 1, Edition Wiley, 2012, p 95) :

$$\frac{D_p}{D_0} = \frac{\varepsilon_p}{\tau} \qquad \text{Équation 1}$$

Une valeur élevée de porosité $\varepsilon_p$, de type macroporosité et/ou mésoporosité, favorise ainsi la diffusion. Cependant, il n'est pas souhaitable d'augmenter fortement la macroporosité et/ou mésoporosité, car cette porosité ne participe pas à la capacité d'adsorption. Par conséquent, l'homme du métier ne cherchera pas à l'augmenter dans le but de réduire le temps de diffusion intra-particulaire dans les macropores et mésopores de l'espace inter-cristallin, sachant que cela se ferait au détriment de la capacité d'adsorption volumique.

Le facteur de tortuosité $\tau$ est une propriété de l'adsorbant et ne dépend pas de la molécule diffusante. Ce facteur $\tau$ permet de rendre compte de la longueur de diffusion lorsque les molécules diffusent à travers un espace poreux tridimensionnel constitué de pores interconnectés entre eux par des restrictions, par rapport à des pores cylindriques rectilignes de même diamètre moyen de pore. Kärger précise (ibid., page 96) que les valeurs de facteur de tortuosité $\tau$ mesurées expérimentalement sont typiquement comprises entre 2 et 5.

Des propriétés de diffusion optimales (c'est-à-dire un transfert de matière optimal), des capacités d'adsorption optimales, tout en conservant une résistance mécanique maximale des adsorbants à structure zéolithique sous forme d'agglomérés ont été obtenues en sélectionnant de manière spécifique à la fois la porosité et le facteur de tortuosité.

### Objet de l'invention

La présente invention a ainsi pour premier objet de proposer des adsorbants zéolithiques sous forme d'agglomérés aux propriétés optimisées pour la séparation de mélanges gazeux ou liquides d'isomères et plus particulièrement pour la séparation des xylènes, en phase gaz ou en phase liquide, notamment du para-xylène des coupes aromatiques en C8. Les adsorbants zéolithiques de l'invention présentent notamment des propriétés maximales de sélectivité vis-à-vis du para-xylène et de transfert de matière, tout en présentant une résistance améliorée et une capacité d'adsorption par volume d'adsorbant élevées et sont particulièrement adaptés pour une utilisation dans un procédé de séparation du para-xylène en phase liquide, de préférence de type contre-courant simulé.

### Résumé de l'invention

L'invention concerne un adsorbant zéolithique sous forme d'agglomérés, selon la revendication 1, ledit adsorbant ayant :

- un facteur de tortuosité $\tau$, calculé à partir de la distribution poreuse déterminée par porosimétrie par intrusion de mercure, strictement supérieur à 1 et strictement inférieur à 3;

- une porosité $\varepsilon_p = \dfrac{V_{ma} + V_{me}}{V_g}$, déterminée par porosimétrie par intrusion de mercure, où $V_{ma}$ désigne le volume macroporeux, $V_{me}$ le volume mésoporeux et $V_g$, le volume de grain, comprise entre 25% et 35%, les volumes étant exprimés en $cm^3.g^{-1}$

De préférence, le facteur de tortuosité $\tau$ est compris entre 1,5 et 2,7.

La résistance mécanique à l'écrasement en lit (REL) élevée, mesurée par la méthode Shell série SMS1471-74 adaptée pour des agglomérés de taille inférieure à 1,6 mm, est généralement supérieure ou égale à 1,0 MPa.

Ledit adsorbant présente avantageusement une taille comprise entre 0,1 mm et 1 mm, bornes incluses.

Ledit adsorbant peut comprendre une zéolithe choisie parmi les zéolithes de structure FAU, de préférence la zéolithe X, seule ou en mélange avec d'autres zéolithes.

Ledit adsorbant peut comprendre plus de 90% en poids de zéolithe(s).

Ladite ou lesdites zéolithe(s) se présentent avantageusement sous la forme de cristaux de taille comprise entre 10 nm et 1500 nm. De manière préférée, la distribution poreuse satisfait aux inéquations a) et/ou b) suivantes :

a)

$$\frac{V_{me}}{V_{me} + V_{ma}} \leq 0,1,$$

b)

$$0,4 \leq \frac{V_{mi}}{V_{ma} + V_{me} + V_{mi}}.$$

$V_{mi}$ désignant le volume microporeux exprimé en $cm^3.g^{-1}$, déterminé par adsorption d'azote.

L'adsorbant zéolithique peut comprendre en outre du baryum et/ou du potassium.

L'invention concerne également un procédé de préparation dudit adsorbant zéolithique comprenant au moins les étapes suivantes:

a) une étape de mélange de cristaux d'au moins une zéolithe, avec un liant d'agglomération contenant au moins 80%, de préférence au moins 90%, de préférence encore au moins 95% en poids d'argile zéolithisable, et éventuellement une source de silice, suivie d'une mise en forme et d'une cuisson des agglomérés à une température comprise entre 500 et 700°C ;

b1) une première étape de zéolithisation par mise en contact des agglomérés zéolithiques obtenus à l'étape a) avec une solution basique alcaline, de concentration comprise entre 0,2 M et 0,9 M, bornes incluses

b2) une deuxième étape de zéolithisation par mise en contact des agglomérés zéolithiques obtenus à l'étape b1) avec une solution basique alcaline, de concentration comprise entre 1,2 M et 4,0 M, bornes incluses

b1 et b2 pouvant être effectuées dans un ordre quelconque et b1 et/ou b2 pouvant être réitérées,

c) une étape éventuelle d'échange cationique des cations contenus dans le milieu réactionnel issu des étapes de zéolithisation par mise en contact avec une solution d'ions baryum, ou d'ions baryum et d'ions potassium,

d) une étape de lavage et séchage des agglomérés zéolithiques ainsi obtenus, et

e) une étape d'activation par chauffage à une température comprise entre 100°C et 400°C, de l'adsorbant zéolithique sous forme d'agglomérés obtenus à l'étape d).

Le procédé peut comprendre une ou plusieurs étapes supplémentaires de mise en forme effectuées après l'une quelconque des étapes a), b1)/b2), c), d), e).

L'invention concerne également un procédé de séparation de *para*-xylène à partir de coupes d'isomères d'hydrocarbures aromatiques contenant 8 atomes de carbone, en phase liquide, par adsorption du *para*-xylène au moyen dudit adsorbant ou d'un adsorbant susceptible d'être préparé selon le procédé de préparation en présence d'un désorbant.

L'invention concerne également un procédé de séparation de para-xylène à partir de coupes d'isomères d'hydrocarbures aromatiques contenant 8 atomes de carbone, en phase gazeuse, par adsorption du para-xylène au moyen dudit adsorbant ou d'un adsorbant susceptible d'être préparé selon le procédé de préparation en présence d'un désorbant.

Ledit procédé de séparation peut être de type lit mobile simulé.

## Description détaillée de l'invention

L'adsorbant zéolithique sous forme d'agglomérés de l'invention comprend à la fois des macropores, des mésopores et des micropores. Par « macropores », on entend des pores dont l'ouverture est supérieure à 50 nm. Par « mésopores », on entend des pores dont l'ouverture est comprise entre 2 nm et 50 nm, bornes non incluses. Par « micropores », on entend des pores dont l'ouverture est inférieure à 2 nm, typiquement supérieure strictement à 0 et inférieure ou égale à 2 nm.

Dans la description de la présente invention, on entend par « distribution poreuse » la répartition du volume poreux en fonction du diamètre des pores.

Par ailleurs, on désigne par « Vma » le volume macroporeux exprimé en $cm^3.g^{-1}$ d'adsorbant, par « Vme » le volume mésoporeux exprimé en $cm^3.g^{-1}$ d'adsorbant et par « Vmi » le volume microporeux exprimé en $cm^3.g^{-1}$ d'adsorbant.

« Vg » représente le volume de grain de l'adsorbant exprimé en $cm^3.g^{-1}$ d'adsorbant.

Plus précisément, la présente invention concerne un adsorbant zéolithique sous forme d'agglomérés, ledit adsorbant ayant :

- un facteur de tortuosité $\tau$, calculé à partir de la distribution poreuse déterminée par porosimétrie par intrusion de mercure, strictement supérieur à 1 et strictement inférieur à 3, de préférence compris entre 1,5 et 2,7 ;

- une porosité $\varepsilon_p = \dfrac{Vma + Vme}{Vg}$ , déterminée par porosimétrie par intrusion de mercure, où Vma désigne le volume macroporeux, Vme le volume mésoporeux et Vg, le volume de grain, comprise entre 25% et 35%, les volumes étant exprimés en $cm^3.g^{-1}$.

Dans la description de l'invention, on entend par « taille » le diamètre moyen en nombre d'un objet, ou sa plus grande dimension moyenne en nombre lorsqu'il n'est pas sphérique. L'adsorbant zéolithique sous forme d'agglomérés de l'invention présente avantageusement une taille typiquement comprise entre 0,1 mm et 1 mm, de préférence comprise entre 0,2 mm et 1 mm, et en particulier comprise entre 0,3 mm et 0,8 mm, plus généralement entre 0,4 mm et 0,7 mm, bornes incluses.

L'adsorbant zéolithique sous forme d'agglomérés selon la présente invention présente avantageusement une résistance mécanique à l'écrasement en lit (REL) élevée, mesurée par la méthode Shell série SMS1471-74 adaptée pour des agglomérés de taille inférieure à 1,6 mm, comme décrit plus loin dans la description. La REL du adsorbant zéolithique sous forme d'agglomérés de l'invention est généralement supérieure ou égale à 1,0 MPa, plus généralement supérieure ou égale à 1,5 MPa, de préférence supérieure ou égale à 2,0 MPa, typiquement supérieure ou égale à 2,1 MPa.

L'adsorbant selon la présente invention est un matériau zéolithique, indiquant qu'il comprend des cristaux d'au moins une zéolithe, choisie parmi les zéolithes de structure FAU, notamment choisie parmi les zéolithes X et Y. Parmi ces zéolithes, on préfère la zéolithe X, sans exclure les mélanges de zéolithe X avec une ou plusieurs des autres zéolithes listées ci-dessus. Le rapport atomique Si/Al des cristaux de zéolithe(s) est avantageusement compris entre 1,00 et 1,50, de préférence entre 1,05 et 1,50, et de manière encore plus préférée compris entre 1,10 et 1,50, bornes incluses mesuré par analyse chimique par fluorescence de rayons X.

De manière préférée, l'adsorbant zéolithique de la présente invention ne comprend qu'une seule forme zéolithique qui est la zéolithe X, identifiée par DRX, de ratio Si/Al compris entre 1,00 et 1,50, de préférence entre 1,05 et 1,50, et de manière encore plus préférée compris entre 1,10 et 1,50, bornes incluses, le ratio étant mesuré par analyse chimique par fluorescence de rayons X.

Avantageusement, l'adsorbant zéolithique sous forme d'agglomérés de l'invention comprend plus de 90% en poids de zéolithe, de manière préférée plus de 94% en poids de zéolithe(s), de manière très préférée plus de 96% en poids de zéolithe(s), de manière encore plus préférée entre 96% et 98% en poids de zéolithe(s), bornes incluses, par rapport au poids total de l'adsorbant zéolithique. Le complément à 100% en poids est constitué par la phase non zéolithique (PNZ) comprenant notamment le liant non converti ou du liant zéolithisé amorphisé, identifiée par DRX.

Selon un mode de réalisation préféré, la (ou les) zéolithe(s) dans l'adsorbant zéolithique de l'invention se présentent sous la forme de cristaux de taille généralement comprise entre 10 nm et 1500 nm, de préférence entre 100 nm et 1200 nm, de préférence encore entre 200 nm et 1100 nm, et de manière tout particulièrement préférée entre 300 nm et 900 nm, bornes incluses.

Dans un mode de réalisation, l'adsorbant zéolithique de la présente invention présente un rapport atomique Si/Al avantageusement compris entre 1,00 et 3,00, de préférence entre 1,00 et 2,00, de préférence encore entre 1,00 et 1,50, bornes incluses, mesuré par analyse chimique par fluorescence de rayons X.

Par ailleurs, l'adsorbant zéolithique selon la présente invention peut contenir un ou plusieurs métaux alcalins et/ou alcalino-terreux, généralement sous forme de cations. Les métaux alcalins et/ou alcalino-terreux présents de préférence sont le sodium, le potassium, le baryum, et les mélanges de deux ou plusieurs d'entre eux en toutes proportions.

L'adsorbant zéolithique sous forme d'agglomérés de la présente invention possède en outre une distribution poreuse spécifique, où les volumes macroporeux et mésoporeux sont mesurés par intrusion de mercure et le volume microporeux est mesuré par adsorption d'azote.

Selon un mode de réalisation tout particulièrement préféré de la présente invention, la distribution poreuse de l'adsorbant zéolithique sous forme d'agglomérés satisfait aux inéquations a) et/ou b) suivantes :

a) $\dfrac{Vme}{Vme + Vma} \leq 0,1$ , de préférence $0,01 \leq \dfrac{Vme}{Vme + Vma} \leq 0,1$ , plus préférentiellement

$0,01 \leq \dfrac{Vme}{Vme + Vma} \leq 0,06$ ;

b) $0,4 \leq \dfrac{Vmi}{Vma + Vme + Vmi}$ , de préférence $0,4 \leq \dfrac{Vmi}{Vma + Vme + Vmi} \leq 0,6$ , de préférence encore $0,45 \leq \dfrac{Vmi}{Vma + Vme + Vmi} \leq 0,55$ .

La faible valeur du facteur de tortuosité de l'aggloméré zéolithique selon la présente invention permet un transport des molécules au travers dudit aggloméré le plus direct possible.

Selon un autre aspect, l'invention concerne également un procédé de préparation des matériaux granulaires zéolithiques tels qu'ils viennent d'être définis, procédé qui comprend au moins les étapes suivantes :

a) mélange de cristaux d'au moins une zéolithe, avec un liant d'agglomération contenant au moins 80%, de préférence au moins 90%, de préférence encore au moins 95% en poids d'argile zéolithisable, et éventuellement une source de silice, mise en forme du mélange obtenu, et cuisson à une température comprise entre 500°C à 700°C, pendant une durée avantageusement comprise entre quelques minutes et quelques heures, de préférence entre 2 et 6 heures ;

b1) une première étape de zéolithisation par mise en contact avec une solution basique alcaline, de concentration comprise entre 0,2 M et 0,9 M, de préférence entre 0,2 M et 0,8 M, bornes incluses

b2) une deuxième étape de zéolithisation par mise en contact avec une solution basique alcaline, de concentration comprise entre 1,2 M et 4,0 M, de préférence entre 1,2 et 3,5, de préférence encore entre 1,5 M et 3,5 M, de manière tout particulièrement préférée entre 1,5 et 3,0, bornes incluses

les étapes b1 et b2 pouvant être effectuées dans un ordre quelconque et b1 et/ou b2 pouvant être réitérées, de préférence chacune des étapes b1 et b2 ne sont mises en œuvre qu'une seule fois,

c) échange cationique éventuel des cations contenu dans le milieu réactionnel issu des étapes de zéolithisation par mise en contact avec une solution d'ions baryum, ou d'ions baryum et d'ions potassium,

d) lavage et séchage des agglomérés zéolithiques ainsi obtenus, et

e) activation par chauffage à une température en général comprise entre 100°C et 400°C, de préférence entre 200°C et 300°C de l'adsorbant zéolithique sous forme d'agglomérés obtenus à l'étape d).

On peut utiliser comme solution basique alcaline pour les étapes de zéolithisation une solution d'hydroxyde de sodium ou d'un mélange d'hydroxyde de sodium et d'hydroxyde de potassium. La zéolithisation se fait en au minimum 2 étapes dont les concentrations sont différentes, la durée de chaque étape étant avantageusement comprise entre quelques secondes et plusieurs heures, de préférence entre 30 secondes et 10 heures, chacune des étapes de zéolithisation pouvant avoir des durées identiques ou différentes, comme explicité plus loin.

Sans vouloir être lié par une quelconque théorie, il semblerait que les deux étapes de zéolithisation avec des solutions basiques alcalines de concentration différente permettent d'obtenir des agglomérés d'adsorbant ayant le facteur de tortuosité et la porosité requis. Les adsorbants obtenus combinent à la fois une capacité d'adsorption maximale et des propriétés améliorées.

Les cristaux de zéolithe(s) mis en œuvre dans le cadre de la présente invention présentent de préférence une taille supérieure ou égale à 100 nm et inférieure ou égale à 1500 nm, de préférence supérieure ou égale à 150 nm et strictement inférieure à 1200 nm, et mieux encore supérieure ou égale à 150 nm et inférieure ou égale à 1100 nm.

Comme indiqué précédemment, on préfère utiliser des cristaux de zéolithe de structure faujasite de rapport atomique Si/Al compris entre 1,00 et 1,50, de préférence entre 1,05 et 1,50, et de manière encore plus préférée compris entre 1,10 et 1,50, bornes incluses mesuré par analyse chimique par fluorescence de rayons X.

Lors de l'étape a), outre les cristaux de zéolithe(s) et le liant d'agglomération, un ou plusieurs additifs peuvent également être ajoutés, par exemple des additifs tels que de la silice, notamment dans le cas où la zéolithe mise en œuvre est une zéolithe X. La source éventuelle de silice peut être de tout type connu de l'homme du métier, spécialiste de la synthèse de zéolithes, par exemple de la silice colloïdale, des diatomées, de la perlite, des cendres de calcination (« fly ash » en langue anglaise), du sable, ou toute autre forme de silice solide.

Les cristaux de zéolithe mis en œuvre à l'étape a) peuvent ainsi être avantageusement issus de la synthèse de cristaux de zéolithe X comprenant majoritairement, voir exclusivement des cations sodium, comme c'est le cas par exemple pour les zéolithes NaX (ou 13X), mais on ne sortirait pas du cadre de l'invention en utilisant des cristaux ayant subi un ou plusieurs échanges cationiques, entre la synthèse sous forme NaX et sa mise en œuvre à l'étape a). Dans ce cas, l'étape d) d'échange cationique devient par conséquent non nécessaire.

L'opération de mise en forme peut faire appel à un liant d'agglomération. Selon un mode de réalisation préféré de l'invention ledit liant comprend au moins 80% d'une argile ou d'un mélange d'argiles, éventuellement zéolithisable(s), et avec jusqu'à 5% d'additifs connus de l'homme du métier tels que par exemple et de manière non limitative : carboxyméthylcellulose, silice, alumine, et autres, ainsi qu'avec la quantité d'eau permettant la mise en forme du matériau aggloméré. L'adsorbant zéolithique sous forme d'agglomérés peut ainsi être mis sous forme de bille, d'extrudé ou autre, de taille comprise entre 0,1 mm et 1 mm, comme indiqué précédemment.

Le liant d'agglomération mis en œuvre à l'étape a) contient au moins 80%, de préférence au moins 90%, de préférence encore au moins 95%, en poids, d'argile ou de mélange d'argiles parmi les kaolins, kaolinites, nacrites, dickites, halloysite et/ou métakaolins et peut contenir jusqu'à 20%, de préférence jusqu'à 10%, de préférence encore jusqu'à 5%, en poids, d'un ou plusieurs additifs et/ou d'un ou plusieurs autres liants minéraux tels que bentonite, attapulgite, sépiolite et autres.

Les proportions de liant d'agglomération et de zéolithe(s) mises en œuvre à l'étape a), et éventuellement à une ou plusieurs autres étapes ultérieures sont comprises entre 5 parties à 20 parties en poids de liant d'agglomération pour 95 parties à 80 parties en poids de zéolithe(s).

Dans tous les cas, les argiles peuvent être utilisées dans leur état brut ou peuvent être préalablement soumises à un ou plusieurs traitements, par exemple choisis parmi calcination, traitement à l'acide, modification chimique, et autres.

Comme indiqué précédemment, on opère, avant les étapes de zéolithisation, une cuisson (ou encore « calcination ») du mélange obtenu à l'étape a) à une température en général comprise entre 500°C et 700°C. Le principe en est exposé dans « Zeolite Molecular Sieves » de D.W. Breck, John Wiley and Sons, New York, (1973), pp. 314-315.

Les étapes de zéolithisation b1 et b2 par mise en contact avec une solution basique alcaline sont typiquement réalisées par immersion du mélange obtenu à l'étape a) dans une solution basique alcaline, en général aqueuse, par exemple une solution aqueuse d'hydroxyde de sodium et/ou d'hydroxyde de potassium, dont les concentrations sont différentes, telles que définies précédemment.

Les étapes de zéolithisation s'opèrent à froid ou à chaud, de préférence à chaud, à une température supérieure à la température ambiante, et typiquement entre la température ambiante (soit environ 20°C) et la température d'ébullition de la solution alcaline, typiquement entre la température ambiante et 100°C et de préférence comprise entre 60°C et 100°C et de manière encore préférée entre 80 et 100°C.

La durée de chaque étape de zéolithisation est généralement comprise entre quelques secondes et quelques heures. De manière générale, on préfère, pour l'étape de zéolithisation à faible concentration (étape b1 comme indiqué précédemment), une durée comprise entre quelques dizaines de minutes à quelques heures, par exemple comprise entre 15 minutes et 8 heures, typiquement comprise entre 30 minutes et 6 heures. Pour l'étape de zéolithisation à forte concentration (étape b2 comme indiqué précédemment), une durée comprise entre quelques secondes à quelques dizaines de minutes, par exemple comprise entre 30 secondes et 2 heures.

Chacune des étapes de zéolithisation est réalisée sous agitation ou en l'absence d'agitation.

On préfère cependant réaliser l'une ou l'autre, voire les deux étapes de zéolithisation, sous agitation afin de maintenir homogène le milieu réactionnel.

Le produit intermédiaire obtenu entre chacune des étapes de zéolithisation peut être isolé ou non. De préférence, le produit intermédiaire n'est pas isolé : les deux étapes de zéolithisation sont conduites l'une après l'autre sans isoler le produit intermédiaire.

L'étape c) d'échange éventuel des cations s'effectue selon les méthodes classiques connues de l'homme du métier, et le plus souvent par mise en contact des agglomérés issus de l'étape b) avec un sel, tel qu'un chlorure, par exemple le chlorure de baryum ($BaCl_2$) pour l'échange au baryum et/ou le chlorure de potassium (KCl) pour l'échange au potassium, en solution aqueuse à une température comprise entre la température ambiante et 100°C, et de préférence comprise entre 80°C et 100°C.

L'activation qui suit le séchage, est conduite de manière classique, selon les méthodes connues de l'homme du métier, par exemple à une température en général comprise entre 100°C et 400°C, de préférence entre 200°C et 300°C. Cette étape f) d'activation a pour but de fixer la teneur en eau, ainsi que la perte au feu de l'adsorbant de façon optimale pour l'utilisation envisagée. On procède en général par activation thermique qu'on exécute préférentiellement entre 200°C et 300°C pendant une durée déterminée en fonction de la teneur en eau et de la perte au feu souhaitées, typiquement de 1 à 6 heures.

Le procédé de l'invention comprend une ou plusieurs étapes de mise en forme qui peu(ven)t être réalisée(s) selon toutes les techniques connues de l'homme du métier, telles que par exemple extrusion, compactage, agglomération, et autres. On préfère effectuer une seule étape de mise en forme au cours de l'étape a) avant la cuisson. De manière optionnelle, une ou plusieurs étapes de mise en forme supplémentaire(s) peuvent être effectuées après l'une ou l'autre des étapes a), b), c), d), e)

L'adsorbant zéolithique sous forme d'agglomérés selon l'invention est particulièrement adapté pour les procédés de séparation de composés en phase liquide, et notamment pour les procédés dans lesquels ledit matériau est soumis à des contraintes mécaniques importantes, par exemple les procédés de séparation en phase liquide à co-courant ou à contre-courant, et plus particulièrement les procédés de séparation en phase liquide en lit mobile simulé. L'adsorbant zéolithique sous forme d'agglomérés selon l'invention est tout particulièrement adapté pour les procédés de séparation des xylènes en phase liquide. L'adsorbant zéolithique selon l'invention peut également être utilisé :

- pour la séparation d'alcools polyhydriques,
- pour la séparation d'isomères de toluène substitué,
- pour la séparation des crésols.

Ainsi, et selon encore un autre aspect, la présente invention concerne l'utilisation d'au moins un adsorbant zéolithique sous forme d'agglomérés tel qu'il vient d'être défini, comme matériau adsorbant dans les procédés de séparation en phase liquide ou gazeuse, à co-courant ou à contre-courant, et plus particulièrement dans les procédés de séparation en phase liquide en lit mobile simulé, typiquement dans les procédés de séparation des coupes aromatiques comportant des mélanges d'isomères aromatiques à 8 atomes de carbone et plus particulièrement dans les procédés en phase liquide de séparation des xylènes en lit mobile simulé, seul ou en couplage avec une unité de cristallisation, et tout particulièrement dans les procédés de récupération de *para-xylène* de haute pureté à partir de coupes d'isomères aromatiques à 8 atomes de carbone.

Enfin, l'invention concerne également le procédé de séparation des coupes aromatiques comportant des mélanges d'isomères à 8 atomes de carbone en phase liquide ou gazeuse. L'invention concerne plus particulièrement le procédé en phase liquide de séparation des xylènes en lit mobile simulé, seul ou en couplage avec une unité de cristallisation, et tout particulièrement le procédé de récupération de *para-xylène* de haute pureté à partir de coupes d'isomères aromatiques à 8 atomes de carbone, comme par exemple décrit dans la demande WO2009/081024, et dans lequel est mis en œuvre au moins un adsorbant zéolithique sous forme d'agglomérés tel que décrit précédemment. Le procédé est mis en œuvre en présence d'un désorbant, de préférence choisi parmi le toluène et le *para*-diéthylbenzène.

L'invention concerne également le procédé en phase gazeuse de séparation des xylènes en lit mobile simulé, par adsorption du *para*-xylène au moyen d'un adsorbant tel que décrit précédemment en présence d'un désorbant, de préférence choisi parmi le toluène et le *para*-diéthylbenzène. De préférence, le procédé est mis en œuvre en lit mobile simulé, de manière très préférée à contre-courant simulé.

## Techniques de caractérisation

Le taux de phase non zéolithique, par exemple liant résiduel non zéolithisé ou toute autre phase amorphe, après zéolithisation est calculé selon l'équation suivante : PNZ = 100 - $\Sigma$ (PZ),

PZ représente la somme des quantités des fractions zéolithiques X au sens de l'invention. La quantité des fractions zéolithiques dans l'adsorbant zéolithique sous forme d'agglomérés est mesurée par analyse par diffraction de rayons X, connue de l'homme du métier sous l'acronyme DRX. Cette analyse est réalisée sur un appareil de la marque Bruker, puis la quantité des fractions zéolithiques PZ et leur somme $\Sigma$ (PZ), sont évaluées au moyen du logiciel TOPAS de la société Bruker. La phase non zéolithique, PNZ, est également évaluée par différence : PNZ = 100 - $\Sigma$ (PZ).

La taille des cristaux de zéolithe(s) à l'étape a) et des cristaux de zéolithe(s) dans les adsorbants est mesurée par observation au microscope électronique à balayage (MEB), en effectuant un ensemble de clichés à un grossissement d'au moins 5000. On mesure ensuite le diamètre d'au moins 200 cristaux à l'aide d'un logiciel d'analyse d'image dédié. La précision est de l'ordre de 3%.

Les cristaux de zéolithe, tels que ceux mis en œuvre à l'étape a) du procédé selon l'invention, ainsi que l'adsorbant zéolithique sous forme d'agglomérés, sont évalués quant au rapport atomique Si/Al et au taux d'échange cationique par analyse chimique élémentaire de l'adsorbant zéolithique, et plus précisément par analyse chimique par fluorescence de rayons X telle que décrite dans la norme NF EN ISO 12677 : 2011 sur un spectromètre dispersif en longueur d'onde (WDXRF), par exemple Tiger S8 de la société Bruker.

Le spectre de fluorescence X a l'avantage de dépendre très peu de la combinaison chimique de l'élément, ce qui offre une détermination précise, à la fois quantitative et qualitative. On obtient de manière classique après étalonnage pour chaque oxyde $SiO_2$ et $Al_2O_3$, ainsi que oxydes de sodium, potassium et baryum, une incertitude de mesure inférieure à 0,4% en poids. L'incertitude de mesure du rapport atomique Si/Al est de $\pm$ 5%.

La détermination de la taille de l'adsorbant est effectuée par analyse de la distribution granulométrique d'un échantillon d'aggloméré par imagerie selon la norme ISO 13322-2:2006, en utilisant un tapis roulant permettant à l'échantillon de passer devant l'objectif de la caméra. La taille de l'objet (diamètre moyen en nombre) est ensuite calculée à partir de la distribution granulométrique en appliquant la norme ISO 9276-2:2001. La précision est de l'ordre de 0,01 mm pour la gamme de taille de l'adsorbant zéolithique sous forme d'agglomérés de l'invention.

Le volume microporeux $V_{mi}$ est déterminé selon toutes méthodes connues de l'homme du métier, par exemple à partir de la mesure de l'isotherme d'adsorption d'un gaz à sa température de liquéfaction, par exemple azote, argon, oxygène, et autres. De préférence, l'azote est utilisé. Préalablement à cette mesure d'adsorption, l'adsorbant zéolithique sous forme d'agglomérés de l'invention est dégazé entre 300°C et 450°C pendant une durée de 9 heures à 16 heures, sous vide (P < 6,7.10$^{-4}$ Pa). Par exemple, pour une zéolithe de structure FAU, la mesure de l'isotherme d'adsorption d'azote à 77K est ensuite effectuée sur un appareil de type ASAP 2020 de Micromeritics, en prenant au moins 35 points de mesure à des pressions relatives de rapport $P/P_0$ compris entre 0,002 et 1. Le volume microporeux est déterminé selon l'équation de Dubinin et Raduskevitch à partir de l'isotherme obtenu, en appliquant la norme ISO 15901-3:2007. Le volume microporeux ainsi évalué s'exprime en cm$^3$ d'adsorbant liquide par gramme d'adsorbant anhydre. L'incertitude de mesure est de $\pm$ 0,003 g/cm$^3$.

Les volumes macroporeux $V_{ma}$ et mésoporeux $V_{me}$, la densité de grain $d_g$ et la porosité $\varepsilon_p$, de type macroporosité et mésoporosité, sont mesurés par porosimétrie par intrusion de mercure. Un porosimètre à mercure type Autopore® 9500 de Micromeritics est utilisé pour analyser la répartition du volume poreux contenu dans les macropores et dans les mésopores.

La méthode expérimentale, décrite dans le manuel opératoire de l'appareil faisant référence à la norme ASTM D4284-83, consiste à placer un échantillon d'adsorbant (adsorbant zéolithique sous forme d'agglomérés à mesurer) (de perte au feu connue) préalablement pesé, dans une cellule du porosimètre, puis, après un dégazage préalable (pression d'évacuation de 30 $\mu$m Hg pendant au moins 10 min), à remplir la cellule avec du mercure à une pression donnée (0,0036 MPa), et ensuite à appliquer une pression croissante par palier jusqu'à 400 MPa afin de faire pénétrer progressivement le mercure dans le réseau poreux de l'échantillon, en prenant au moins 15 paliers de pression jusqu'à 0,2 Mpa, et en appliquant ensuite des incréments de 0,1 MPa jusqu'à 1 MPa, puis 0,5 MPa jusqu'à 10MPa, puis 2 MPa jusqu'à 30 MPa, puis 5 Mpa jusqu'à 180 MPa, et enfin 10 Mpa jusqu'à 400 MPa.

La relation entre la pression appliquée et la dimension caractéristique du seuil d'entrée de pore (correspondant à un diamètre apparent de pore) est établie en utilisant l'équation de Laplace-Young et en supposant une ouverture de pore cylindrique, un angle de contact entre le mercure et la paroi des pores de 140° et une tension de surface du mercure de 485 dynes/cm. Les incréments de volume $\Delta Vi$ de mercure introduit à chaque palier de pression $Pi$ sont enregistrés, ce qui permet ensuite de tracer le volume cumulé de mercure introduit en fonction de la pression appliquée $V(Pi)$, ou en fonction du diamètre apparent des pores $V(li)$. On fixe à 0,2 MPa la valeur à partir de laquelle le mercure remplit tous les vides inter-granulaires, et on considère qu'au-delà le mercure pénètre dans les pores de l'adsorbant. Le volume de grain Vg est alors calculé en soustrayant le volume cumulé de mercure à cette pression (0,2 MPa) au volume de la cellule du porosimètre, et en divisant cette différence par la masse de l'adsorbant équivalent anhydre, c'est-à-dire la masse dudit matériau corrigée de la perte au feu. La densité de grain $dg$ est l'inverse du volume de grain $Vg$ défini précédemment.

Le volume macroporeux $Vma$ de l'adsorbant est défini comme étant le volume cumulé de mercure introduit à une pression comprise entre 0,2 MPa et 30 MPa, correspondant au volume contenu dans les pores de diamètre apparent supérieur à 50 nm. Le volume mésoporeux $Vme$ de l'adsorbant est défini comme étant le volume cumulé de mercure introduit à une pression comprise entre 30 MPa et 400 MPa. La méthode de mesure du volume poreux par intrusion de mercure ne permettant pas d'accéder au volume microporeux, le volume poreux total $V_{tot}$ tel que mesuré par intrusion de mercure, correspond à la somme des volumes macroporeux $V_{ma}$ et mésoporeux $V_{me}$.

Dans le présent document, les volumes macroporeux et mésoporeux $V_{ma}$ et $V_{me}$, ainsi que leur somme (volume poreux total $V_{tot}$), des adsorbants zéolithiques, exprimés en cm$^3$.g$^{-1}$, sont ainsi mesurés par porosimétrie par intrusion de mercure et rapportés à la masse de l'échantillon en équivalent anhydre, c'est-à-dire la masse dudit adsorbant corrigée de la perte au feu. La densité de grain $dg$ est exprimée en g.cm$^{-3}$ et se réfère à la masse de l'échantillon en équivalent anhydre.

La porosité $\varepsilon_p$ de type macroporosité et mésoporosité, est le produit de la densité de grain $d_g$ par la somme des volumes macroporeux et mésoporeux $Vma$ et $Vme$ : $\varepsilon_p = d_g \times (V_{ma} + V_{me})$.

Le facteur de tortuosité $\tau$ est déterminé par une mesure standard détaillée dans le manuel utilisateur de l'Autopore® et modifiée pour le cas des matériaux microporeux. La mesure est basée sur l'équation suivante, issue de Carniglia dans "Construction of the tortuosity factor from porosimetry" J. of Catalysis 102, 401-418 (1986)) :

$$\tau = \left(2.23 - 1,13 \cdot V_{tot} \cdot d_g\right)\left(0,92 \frac{4}{S_S} \sum_{Pi=0,2MPa}^{Pdc} \frac{\Delta Vi}{li}\right)^{1+\delta} \qquad \text{équation 2}$$

Les paramètres $\delta$ et $S_s$ sont deux paramètres utilisateurs :

- $\delta$ est un exposant traduisant le mécanisme de diffusion : il est égal à 0 ou 1 selon que l'on évalue les propriétés de transport des adsorbants zéolithiques pour leur utilisation dans des procédés en phase liquide ou en phase gaz respectivement.
- $S_s$ est l'aire de surface des pores de type macropores et mésopores, exprimée en $m^2$ par gramme d'échantillon en équivalent anhydre. Le manuel utilisateur suggère d'affecter au paramètre $S_s$ la valeur de la surface spécifique BET mesurée par porosimétrie par adsorption d'azote. Cependant dans le cas des matériaux selon l'invention, obtenus par agglomération de cristaux individuels de zéolithe, la surface spécifique BET intègre non seulement la surface des pores de type macropores et mésopores, mais également une surface équivalente représentant l'adsorption dans les micropores. Dans la présente invention, le facteur de tortuosité est donc déterminé en utilisant un paramètre utilisateur $S_s$ adapté aux cas des agglomérés à structure zéolithique de la manière suivante : le paramètre $S_s$ peut être assimilé à la surface externe des cristaux constitutifs de l'adsorbant rapportée à leur masse. Pour des cristaux de zéolithes faujasites, pour lesquels le rapport volume sur surface externe est supposé égal au diamètre des cristaux divisé par 6 (même rapport que pour une sphère), le paramètre de surface $S_s$ est calculé selon l'équation 3 suivante :

$$S_S = \frac{6 \cdot V_{mi}}{f \cdot d_{cristaux}} \qquad \text{équation 3,}$$

où f est la fraction de vide dans les cristaux, et $d_{cristaux}$ est le diamètre moyen en nombre des cristaux de zéolithe de l'adsorbant zéolithique mesuré par observation au microscope électronique à balayage (MEB). La valeur de f est prise égale à 0,5 dans le cas d'une zéolithe NaX de rapport Si/Al=1,25 (Table 5.9, p 429 de l'ouvrage « Zeolite Molecular Sieves » de D.W. Breck, John Wiley and Sons, New York, (1973)) et on conservera cette valeur dans le cas d'adsorbant zéolithique après échange des cations sodium avec des cations baryum et/ou potassium, car ce ratio volumique n'est pas modifié lors de l'échange avec ce type de cations.

Dans l'équation 2, la somme est effectuée pour des pressions d'intrusion de mercure comprises entre 0,2MPa et une pression *Pdc* correspondant à un diamètre de seuil d'entrée de pore apparent *ldc* égal à :

- *ldc* = 25 fois le rayon moléculaire des molécules qui seront transportées dans l'adsorbant zéolithique pour une utilisation en séparation de mélange liquide, soit 17nm dans le cadre de l'invention appliquée aux molécules de xylènes, c'est à dire une pression *Pdc* égale à 87 MPa.
- *ldc* =5 fois le libre parcours moyen du gaz l'adsorbant zéolithique pour une utilisation en séparation de mélange gazeux.

La résistance mécanique mesurée est la résistance à l'écrasement en lit (REL) caractérisée selon la méthode Shell série SMS1471-74 (Shell Method Series SMS1471-74 « Determination of Bulk Crushing Strength of Catalysts. Compression-Sieve Method »), associée à l'appareil « BCS Tester » commercialisé par la société Vinci Technologies.

Cette méthode de mesure de la REL, initialement destinée à la caractérisation de catalyseurs de 3 mm à 6 mm est basée sur l'utilisation d'un tamis de 425 $\mu$m qui va permettre notamment de séparer les fines créées lors de l'écrasement. L'utilisation d'un tamis de 425 $\mu$m reste adaptée pour des particules de diamètre supérieur à 1,6 mm, mais doit être adaptée selon la granulométrie du matériau que l'on cherche à caractériser. Pour l'adsorbant zéolithique sous forme d'agglomérés de la présente invention, un tamis de 200 $\mu$m est utilisé à la place du tamis de 425 $\mu$m mentionné dans la méthode Shell standard SMS1471-74.

Le protocole de mesure est le suivant : un échantillon de 20 cm³ de matériau à analyser, préalablement tamisé avec le tamis adapté (200 $\mu$m) et préalablement séché à l'étuve pendant au moins 2 heures à 250°C (au lieu de 300°C mentionné dans la méthode Shell standard SMS1471-74), est placé dans un cylindre métallique de section interne connue. Une force croissante est imposée par paliers sur cet échantillon par l'intermédiaire d'un piston, à travers un lit de 5 cm³ de billes d'acier afin de mieux répartir la force exercée par le piston sur le matériau (utilisation de billes de 2 mm de diamètre pour des particules de forme sphérique de diamètre strictement inférieur à 1,6 mm). Les fines obtenues aux différents paliers de pression sont séparées par tamisage (tamis adapté de 200 μm) et pesées.

La REL est déterminée par la pression en mégaPascal (MPa) pour laquelle la quantité de fines cumulées passant à travers le tamis s'élève à 0,5% pondéral de l'échantillon. Cette valeur est obtenue en traçant sur un graphique la masse de fines obtenue en fonction de la force appliquée sur le lit de matériau zéolithique et en interpolant à 0,5 % massique de fines cumulées. La résistance mécanique à l'écrasement en lit est typiquement comprise entre quelques centaines de kPa et quelques dizaines de MPa et généralement comprise entre 0,3 MPa et 3,2 MPa. La précision est de manière classique inférieure à 0,1 MPa.

Les exemples suivants permettent d'illustrer l'objet de l'invention. Ils sont fournis à titre indicatif seulement, et ne sont destinés en aucune façon à limiter les divers modes de réalisation de la présente invention.

**Exemple A : Préparation des adsorbants zéolithiques à base de zéolithe X**

Synthèse de cristaux de zéolithe X, de rapport atomique Si/Al = 1,25, de diamètre moyen en nombre de 1,0 μm et de rapport atomique Na/Al = 1.

On prépare un gel de composition molaire 3,5 $Na_2O$ - 2,8 $SiO_2$ - $Al_2O_3$ - 130 H2O par mélange des réactifs suivants : silicate de sodium, aluminate de sodium et eau. Le gel est mûri à 35°C pendant 20 heures, et on opère une cristallisation pendant 4 heures à 100°C.

Les cristaux obtenus après filtration et lavage sont identifiés par diffraction des rayons X (analyse DRX) comme étant des cristaux de faujasite. L'analyse chimique du solide donne un rapport atomique Si/Al = 1,25. Le volume microporeux évalué d'après l'équation de Dubinin- Raduskevitch tel que décrit dans la partie technique de caractérisation et exprimé en $cm^3$ par gramme d'adsorbant sec est de 0,345 ± 0,003 $cm^3$/g. L'analyse de la taille des cristaux de zéolithe est réalisée par microscopie électronique à balayage et montre que leur diamètre moyen en nombre ($d_{cristaux}$) est de 1,0 μm.

Préparation des adsorbants zéolithiques

On prépare un mélange homogène et on agglomère 800 g de cristaux de zéolithe NaX préparée selon le mode opératoire décrit ci-dessus, avec 105 g de kaolin (exprimés en équivalent calciné) et 45 g de silice colloïdale vendue sous la dénomination commerciale Klebosol™ 30N50 (contenant 30% en poids de $SiO_2$ et 0,5% en poids de $Na_2O$) avec la quantité d'eau qui permet l'extrusion du mélange. Les extrudés sont séchés, puis calcinés à 550°C (cuisson de l'argile) sous courant d'azote pendant 2 heures, et enfin concassés de manière à récupérer des agglomérats dont le diamètre moyen en nombre est égal à 0,5 mm.

**Exemple 1 (comparatif) :**

20 g d'agglomérats obtenus comme décrit ci-dessus à partir des cristaux de zéolithe X synthétisés dans l'exemple A sont placés dans un réacteur en verre muni d'une double enveloppe régulée à une température de 100°C ± 1°C puis on ajoute 150 mL d'une solution aqueuse d'hydroxyde de sodium de concentration de 2,5 M, et on laisse le milieu réactionnel sous agitation pendant une durée de 4 à 5 heures.

On procède ensuite au lavage des agglomérats en 3 opérations successives de lavage à l'eau suivi de la vidange du réacteur. On s'assure de l'efficacité du lavage en mesurant le pH final des eaux de lavage qui est compris entre 10,0 et 10,5.

Ces agglomérats sont échangés, par mise en contact avec une solution de chlorure de baryum 0,5 M à 95°C en 4 étapes. À chaque étape, le rapport volume de solution sur masse de solide est de 20 mL/g et l'échange est poursuivi pendant 4 heures à chaque fois. Entre chaque échange, le solide est lavé plusieurs fois de manière à le débarrasser des excédents de sel. Les agglomérats sont ensuite séchés à 80°C pendant 2 heures et enfin activés à 250°C pendant 2 heures sous courant d'azote.

La distribution poreuse et la résistance mécanique (REL) des agglomérats sont caractérisées par les techniques de caractérisation décrites ci-dessus. Les résultats sont donnés dans le tableau 1 ci-dessous. Les valeurs de la porosité $\varepsilon_p$, calculée selon l'équation 2 en prenant δ=0, et du facteur de tortuosité τ sont données dans le tableau 2 ci-dessous.

Les taux d'échange baryum des agglomérats calculés à partir des analyses élémentaires des oxydes de baryum et de sodium par fluorescence de rayons X, comme décrit dans les techniques de caractérisation, est de 99,6 ± 0,2%. La perte au feu mesurée comme décrit précédemment est de 5,2% ± 0,1%.

**Exemple 2 (selon l'invention)** :

**1ère étape** :

20 g d'agglomérats obtenus à partir de la poudre synthétisée dans l'exemple A sont placés dans un réacteur en verre muni d'une double enveloppe régulée à une température de 100°C ± 1°C, puis on ajoute 100 mL d'une solution aqueuse d'hydroxyde de sodium de concentration 0,5 M et on laisse le milieu réactionnel sous agitation pendant 3 heures.

**2nde étape** :

Au bout de 3 heures, on ajoute à cette solution, 100 mL d'une solution d'hydroxyde de sodium 5,5 M préparée préalablement et maintenue à une température de 100°C ± 1°C, afin d'obtenir une concentration en hydroxyde de sodium égale à 3M dans le milieu réactionnel. On laisse le milieu réactionnel sous agitation pendant 1 heure.

On procède ensuite au lavage des agglomérats en 3 opérations successives de lavage à l'eau suivi de la vidange du réacteur. On s'assure de l'efficacité du lavage en mesurant le pH final des eaux de lavage qui reste généralement compris entre 10,0 et 10,5.

Ces agglomérats sont engagés dans une réaction d'échange cationique par mise en contact avec une solution aqueuse de chlorure de baryum 0,5 M à 95°C en 4 étapes. À chaque étape, le rapport volume de solution sur masse de solide est de 20 mL/g et l'échange est poursuivi pendant 4 heures à chaque fois. Entre chaque échange, le solide est lavé plusieurs fois de manière à le débarrasser des excédents de sel, par exemple 4 lavages de 50mL d'eau. Les agglomérats sont ensuite séchés à 80°C pendant 2 heures et enfin activés à 250°C pendant 2 heures sous courant d'azote.

Les produits sont caractérisés afin de déterminer par toutes les techniques analytiques décrites ci-dessus : le volume de Dubinin-Raduskevitch est de 0,260 cm$^3$/g et la REL de 3,1 MPa.

La perte au feu mesurée, comme décrit précédemment, est de 5,2% ± 0,1% pour chaque échantillon. Les taux d'échange baryum des agglomérés calculés à partir des analyses élémentaires des oxydes de baryum et de sodium par fluorescence de rayons X, comme décrit dans les techniques de caractérisation, est de 99,4 ± 0,2%.

Tableau 1

| | REL (MPa) | Vg (c$_m$$^3$/g) | Vmi (c$_m$$^3$/g) | Vme (cm$^3$/g$_)$ | Vma (cm$^3$/g$_)$ | Vme/ (Vme+Vma) | Vmi/ (Vmi+Vme+ Vma) |
|---|---|---|---|---|---|---|---|
| Exemple 1 (comparatif) | 2,62 | 0,840 | 0,258 | 0,014 | 0,261 | 5,1% | 48% |
| Exemple 2 (selon l'invention) | 3,10 | 0,800 | 0,260 | 0,008 | 0,212 | 3,5% | 55% |

Tableau 2

| | Paramètre de surface S$_s$ de l'équation 2 (calculé par équation 3 avec f=0,5) | Facteur de tortuosité τ (équation 2 avec δ=0 et Pdc=87MPa) | Porosité εp |
|---|---|---|---|
| Exemple 1 (comparatif) | 3,3 | 3,2 | 32% |
| Exemple 2 (selon l'invention) | 3,5 | 2,1 | 28% |

**Exemple 3** : Test de séparation des xylènes (test en perçage)

Un test de perçage (chromatographie frontale) est ensuite réalisé sur les adsorbants décrits dans les exemples 1 et 2 pour évaluer leur efficacité. La quantité d'adsorbant utilisée pour ces tests est de 72 g.

Le mode opératoire pour obtenir les courbes de perçage est le suivant :

• Remplissage de la colonne par le tamis et mise en place dans le banc de test.

• Remplissage par le solvant (paradiéthylbenzène) à température ambiante.

• Montée progressive à la température d'adsorption sous flux de solvant (5 cm$^3$/min).

• Injection de solvant à 20 cm$^3$/min lorsque la température d'adsorption est atteinte

- Permutation solvant/charge pour injecter la charge (20 cm$^3$/min).

- L'injection de la charge est ensuite maintenue jusqu'à ce que la concentration en solvant dans l'effluent soit nulle, afin d'atteindre l'équilibre thermodynamique.

La pression est suffisante pour que la charge reste en phase liquide, soit 1 MPa. La température d'adsorption est de 175°C.

La composition de la charge est la suivante :

- Paraxylène : 45% poids

- Métaxylène : 45% poids

- Iso-octane : 10% poids (celui-ci est utilisé comme traceur pour l'estimation des volumes non-sélectifs et n'intervient pas dans la séparation)

Tableau 3

| Nature du solide | PAF[1] à 950°C | Capacité [2] | Sélectivité[3] $\alpha_{PX/MX}$ | HEPT iC8 [4] | HEPT PX[5] |
|---|---|---|---|---|---|
| Ex. 1 (comparatif) | 5,2% | 0,211 | 3,57 | 3,3 | 7,1 |
| Ex. 2 | 5,2% | 0,218 | 3,63 | 2,8 | 6,1 |

(1) PAF : Perte au feu
(2) La capacité est exprimée en cm$^3$ de C8-aromatiques adsorbés par gramme d'adsorbant
(3) PX : paraxylène, MX : métaxylène
(4) HEPT iC8 : Hauteur de plateau théorique exprimée en cm du front de perçage de l'iso-octane
(5) HEPT PX : Hauteur de plateau théorique exprimée en cm du front de perçage du paraxylène

Les résultats du tableau 3 montrent que des agglomérés selon l'invention conduisent à une hauteur de plateau théorique moindre et de fait à une productivité meilleure et une pureté meilleure du produit attendu.

**Exemple 4 (comparatif) :**

**1$^{ère}$ étape** :

20 g d'agglomérats obtenus à partir de la poudre synthétisée dans l'exemple A sont placés dans un réacteur en verre muni d'une double enveloppe régulée à une température de 100°C $\pm$ 1°C, puis on ajoute 200 mL d'une solution aqueuse d'hydroxyde de sodium de concentration 0,5 M et on laisse le milieu réactionnel sous agitation pendant 4 heures.

On procède ensuite au lavage des agglomérats en 3 opérations successives de lavage à l'eau suivi de la vidange du réacteur. On s'assure de l'efficacité du lavage en mesurant le pH final des eaux de lavage qui reste généralement compris entre 10,0 et 10,5.

Ces agglomérats sont engagés dans une réaction d'échange cationique par mise en contact avec une solution aqueuse de chlorure de baryum 0,5 M à 95°C en 4 étapes. À chaque étape, le rapport volume de solution sur masse de solide est de 20 mL/g et l'échange est poursuivi pendant 4 heures à chaque fois. Entre chaque échange, le solide est lavé plusieurs fois de manière à le débarrasser des excédents de sel, par exemple 4 lavages de 50mL d'eau. Les agglomérats sont ensuite séchés à 80°C pendant 2 heures et enfin activés à 250°C pendant 2 heures sous courant d'azote.

Les produits sont caractérisés. Le volume de Dubinin-Raduskevitch est de 0,250 cm$^3$/g.

**Exemple 5 (comparatif)** :

**1$^{ère}$ étape** :

20 g d'agglomérats obtenus à partir de la poudre synthétisée dans l'exemple A sont placés dans un réacteur en verre muni d'une double enveloppe régulée à une température de 100°C $\pm$ 1°C, puis on ajoute 200 mL d'une solution aqueuse d'hydroxyde de sodium de concentration 3 M et on laisse le milieu réactionnel sous agitation pendant 4 heures.

On procède ensuite au lavage des agglomérats en 3 opérations successives de lavage à l'eau suivi de la vidange du réacteur. On s'assure de l'efficacité du lavage en mesurant le pH final des eaux de lavage qui reste généralement compris entre 10,0 et 10,5.

Ces agglomérats sont engagés dans une réaction d'échange cationique par mise en contact avec une solution aqueuse de chlorure de baryum 0,5 M à 95°C en 4 étapes. À chaque étape, le rapport volume de solution sur masse de solide est de 20 mL/g et l'échange est poursuivi pendant 4 heures à chaque fois. Entre chaque échange, le solide est lavé plusieurs fois de manière à le débarrasser des excédents de sel, par exemple 4 lavages de 50mL d'eau. Les agglomérats sont ensuite séchés à 80°C pendant 2 heures et enfin activés à 250°C pendant 2 heures sous courant d'azote.

Les produits sont caractérisés. Le volume de Dubinin-Raduskevitch est de 0,240 cm$^3$/g.

Tableau 4

| | Paramètre de surface $S_s$ de l'équation 2 (calculé par équation 3 avec f=0,5) | Facteur de tortuosité $\tau$ (équation 2 avec $\delta$=0 et Pdc=87MPa) | Porosité $\varepsilon$p |
|---|---|---|---|
| Exemple 4 (comparatif) | 3,2 | 3,3 | 36% |
| Exemple 5 (comparatif) | 3,1 | 3,4 | 36% |

## Revendications

1. Adsorbant zéolithique sous forme d'agglomérés, ledit adsorbant ayant :

   - un facteur de tortuosité $\tau$, de formule suivante :

$$\tau = \left(2.23 - 1{,}13 \cdot V_{tot} \cdot d_g\right)\left(0{,}92 \frac{4}{S_S} \sum_{Pi=0{,}2MPa}^{Pdc} \frac{\Delta Vi}{li}\right)^{1+\delta}$$

   dans laquelle

   $V_{tot}$ représente le volume poreux total, c'est-à-dire la somme des volumes macroporeux et mésoporeux $V_{ma}$ et $V_{me}$ exprimés en cm$^3$.g$^{-1}$, $V_{tot}$, $V_{ma}$ et $V_{me}$ étant mesurés par porosimétrie par intrusion de mercure et rapportés à la masse de l'échantillon en équivalent anhydre, $\delta$ = 0 pour la phase liquide ou 1 pour la phase gaz,

   $S_s$ est l'aire de surface des pores de type macropores et mésopores, exprimée en m$^2$ par gramme d'échantillon en équivalent anhydre, calculée selon l'équation 3 suivante :

$$S_S = \frac{6 \cdot V_{mi}}{f \cdot d_{cristaux}}$$

équation 3

   Où f est la fraction de vide dans les cristaux,

   $d_{cristaux}$ est le diamètre moyen en nombre des cristaux de zéolithe de l'adsorbant zéolithique mesuré par observation au microscope électronique à balayage (MEB), et

   $V_{mi}$ désignant le volume microporeux exprimé en cm$^3$.g$^{-1}$, déterminé par adsorption d'azote, le volume microporeux étant déterminé selon l'équation de Dubinin et Raduskevitch à partir de l'isotherme obtenu, en appliquant la norme ISO 15901 - 3:2007, le volume microporeux correspondant au volume des pores ayant une ouverture inférieure à 2 nm,

   $\Delta V_i$ est le volume incrémentiel de mercure, et li est le diamètre apparent des pores,

   dg est la densité de grain exprimée en g.cm$^{-3}$, et se réfère à la masse de l'échantillon en équivalent anhydre,

   Pdc = 87MPa,

   le facteur de tortuosité étant calculé à partir de la distribution poreuse déterminée par porosimétrie par intrusion de mercure, strictement supérieur à 1 et strictement inférieur à 3;

   le facteur de tortuosité étant déterminé par une mesure standard détaillée dans le manuel utilisateur d'un porosimètre à mercure type Autopore® 9500 de Micromeritics;

- une porosité $\varepsilon_p = \dfrac{Vma + Vme}{Vg}$ , déterminée par porosimétrie par intrusion de mercure, où $V_{ma}$ désigne le volume macroporeux, $V_{me}$ le volume mésoporeux et $V_g$, le volume de grain, comprise entre 25% et 35%, les volumes étant exprimés en $cm^3.g^{-1}$ ; le volume macroporeux correspondant au volume des pores ayant une ouverture supérieure à 50 nm, et le volume mésoporeux correspondant au volume des pores ayant une ouverture entre 2 nm et 50 nm bornes non incluses, ledit adsorbant comprenant une zéolithe choisie parmi les zéolithes de structure FAU,
les mesures de porosimétrie par intrusion de mercure étant effectuées selon la norme ASTM D4284-83.

2. Adsorbant zéolithique selon la revendication 1 dans lequel le facteur de tortuosité $\tau$ est compris entre 1,5 et 2,7.

3. Adsorbant zéolitique selon l'une des revendications 1 ou 2 dans lequel la résistance mécanique à l'écrasement en lit (REL) élevée, mesurée par la méthode Shell série SMS1471-74 adaptée pour des agglomérés de taille inférieure à 1,6 mm, est supérieure ou égale à 1,0 MPa, la taille correspondant au diamètre moyen en nombre de l'aggloméré ou à la plus grande dimension moyenne en nombre de l'aggloméré lorsque l'aggloméré n'est pas sphérique.

4. Adsorbant zéolithique selon l'une des revendications 1 à 3 dans lequel ledit adsorbant présente une taille comprise entre 0,1 mm et 1 mm, bornes incluses, la taille correspondant au diamètre moyen en nombre de l'adsorbant ou à la plus grande dimension moyenne en nombre de l'adsorbant lorsque l'adsorbant n'est pas sphérique, la détermination de la taille étant effectuée par analyse de la distribution granulométrique d'un échantillon d'aggloméré par imagerie selon la norme ISO 13322-2:2006, en utilisant un tapis roulant permettant à l'échantillon de passer devant l'objectif de la caméra, la taille de l'objet étant calculée à partir de la distribution granulométrique en appliquant la norme ISO 9276-2 :2001.

5. Adsorbant zéolithique selon l'une des revendications 1 à 4, ladite zéolithe étant la zéolithe X, seule ou en mélange avec d'autres zéolithes.

6. Adsorbant zéolithique selon l'une des revendications 1 à 5, ledit adsorbant comprenant plus de 90% en poids de zéolithe(s).

7. Adsorbant zéolithique selon l'une des revendications 1 à 6, dans lequel ladite ou lesdites zéolithe(s) se présentent sous la forme de cristaux de taille comprise entre 10 nm et 1500 nm, la taille correspondant au diamètre moyen en nombre des cristaux ou à la plus grande dimension moyenne en nombre des cristaux lorsque les cristaux ne sont pas sphériques, la taille étant mesurée par observation au microscope électronique à balayage (MEB).

8. Adsorbant zéolithique selon l'une des revendications 1 à 7 dans lequel la distribution poreuse satisfait aux inéquations a) et/ou b) suivantes :

a)

$$\frac{Vme}{Vme + Vma} \leq 0,1,$$

b)

$$0,4 \leq \frac{Vmi}{Vma + Vme + Vmi}.$$

Vmi désignant le volume microporeux exprimé en $cm^3.g^{-1}$, déterminé par adsorption d'azote, le volume microporeux correspondant au volume des pores ayant une ouverture inférieure à 2 nm.

9. Adsorbant zéolithique selon l'une des revendications 1 à 8 comprenant en outre du baryum et/ou du potassium.

**10.** Procédé de préparation d'un adsorbant zéolithique selon l'une des revendications 1 à 9 comprenant au moins les étapes suivantes:

a) une étape de mélange de cristaux d'au moins une zéolithe, avec un liant d'agglomération contenant au moins 80%, de préférence au moins 90%, de préférence encore au moins 95% en poids d'argile zéolithisable, suivie d'une mise en forme et d'une étape de cuisson à une température comprise entre 500 et 700°C,

b1) une première étape de zéolithisation par mise en contact du matériau obtenu à l'étape a) avec une solution basique alcaline, de concentration comprise entre 0,2 M et 0,9 M, bornes incluses

b2) une deuxième étape de zéolithisation par mise en contact du matériau obtenu à l'étape b1) avec une solution basique alcaline, de concentration comprise entre 1,2 M et 4,0 M, bornes incluses

b1 et b2 pouvant être effectuées dans un ordre quelconque et b1 et/ou b2 pouvant être réitérées,

d) une étape de lavage et séchage du matériau ainsi obtenu, et

e) une étape d'activation du matériau obtenu à l'étape d), par chauffage à une température comprise entre 100°C et 400°C, et récupération de l'adsorbant zéolithique sous forme d'aggloméré.

**11.** Procédé de préparation selon la revendication 10 comprenant une étape c) d'échange cationique des cations contenus dans le milieu réactionnel issu des étapes de zéolithisation par mise en contact avec une solution d'ions baryum, ou d'ions baryum et d'ions potassium, cette étape étant effectuée entre les étapes b2) et d) ou entre les étapes b1) et d).

**12.** Procédé de préparation selon l'une des revendications 10 ou 11 comprenant une ou plusieurs étapes supplémentaires de mise en forme effectuées après l'une quelconque des étapes a), b1), b2), éventuellement c), d), e).

**13.** Procédé de préparation selon l'une des revendications 10 à 12 dans lequel une source de silice est présente dans le mélange de cristaux d'au moins une zéolithe, avec le liant d'agglomération dans l'étape a).

**14.** Procédé de séparation de para-xylène à partir de coupes d'isomères d'hydrocarbures aromatiques contenant 8 atomes de carbone, en phase liquide ou en phase gazeuse, par adsorption du para-xylène au moyen d'un adsorbant selon l'une des revendications 1 à 9 en présence d'un désorbant.

**15.** Procédé selon la revendication 14, de type lit mobile simulé.

**Patentansprüche**

**1.** Zeolithisches Adsorptionsmittel in Form von Agglomeraten, wobei das Adsorptionsmittel Folgendes aufweist:

- einen Tortuositätsfaktor $\tau$ der folgenden Formel:

$$\tau = \left(2.23 - 1.13 \cdot V_{tot} \cdot d_g\right)\left(0.92 \frac{4}{S_S} \sum_{Pi=0.23 \, Pa}^{Pd} \frac{\Delta Vi}{li}\right)^{1-\delta}$$

wobei

$V_{tot}$ das gesamte Porenvolumen darstellt, das heißt die Summe der makroporösen und mesoporösen Volumina $V_{ma}$ und $V_{me}$, ausgedrückt in $cm^3.g^{-1}$, wobei $V_{tot}$, $V_{ma}$ und $V_{me}$ durch Quecksilberintrusionsporosimetrie gemessen und auf die Masse der Probe in wasserfreiem Äquivalent bezogen werden, $\delta = 0$ für die Flüssigphase oder 1 für die Gasphase,

$S_s$ ist die Porenoberfläche des Makro- und Mesoporentyps, ausgedrückt in $m^2$ pro Gramm Probe in wasserfreiem Äquivalent, berechnet gemäß der folgenden Gleichung 3:

$$S_S = \frac{6 \cdot V_{mi}}{f \cdot d_{cristaux}}$$

Gleichung 3

wobei f der Anteil an Vakuum in den Kristallen ist,

$d_{cristaux}$ der zahlenmittlere Durchmesser der Zeolithkristalle des Zeolith-Adsorptionsmittels ist, gemessen durch Beobachtung mit dem Rasterelektronenmikroskop (REM), und

wobei $V_{mi}$ das *mikroporöse* Volumen in $cm^3 \cdot g^{-1}$ bezeichnet, das durch Stickstoffadsorption bestimmt wird, wobei das mikroporöse Volumen gemäß der Gleichung von Dubinin und Raduskevitch anhand der erlangten Isotherme unter Anwendung der Norm ISO 15901 - 3:2007 bestimmt wird, wobei das mikroporöse Volumen dem Volumen der Poren mit einer Öffnung von weniger als 2 nm entspricht, $\Delta V_i$ das inkrementelle Quecksilbervolumen ist, und li der scheinbare Porendurchmesser ist,

dg die Korndichte in $g \cdot cm^{-3}$ ist und sich auf die Masse der Probe in wasserfreiem Äquivalent bezieht, Pdc = 87 MPa,

wobei der Tortuositätsfaktor, der anhand der durch Quecksilberintrusionsporosimetrie bestimmten Porenverteilung berechnet wird, strikt größer als 1 und strikt kleiner als 3 ist;

der Tortuositätsfaktor durch eine standardmäßige Messung bestimmt, die im Benutzerhandbuch für ein Quecksilberporosimeter des Typs Autopore® 9500 von Micromeritics ausführlich beschrieben ist;

- eine Porosität $\varepsilon_p = \dfrac{Vma + Vme}{Vg}$ , bestimmt durch Quecksilberintrusionsporosimetrie, wobei $V_{ma}$ das

makroporöse Volumen, $V_{me}$ das mesoporöse Volumen und $V_g$ das Kornvolumen bezeichnet, zwischen 25 % und 35 %, wobei die Volumina in $cm^3 \cdot g^{-1}$ ausgedrückt sind, wobei das makroporöse Volumen dem Volumen der Poren mit einer Öffnung von mehr als 50 nm entspricht und das mesoporöse Volumen dem Volumen der Poren mit einer Öffnung zwischen 2 nm und 50 nm entspricht, wobei die Grenzwerte nicht eingeschlossen sind, das Adsorptionsmittel umfassend einen Zeolithen, der ausgewählt ist aus den Zeolithen mit FAU-Struktur,

wobei die Quecksilberintrusion-Porosimetriemessungen gemäß ASTM D4284-83 durchgeführt werden.

2. Zeolithisches Adsorptionsmittel nach Anspruch 1, wobei der Tortuositätsfaktor $\tau$ zwischen 1,5 und 2,7 liegt.

3. Zeolithisches Adsorptionsmittel nach einem der Ansprüche 1 oder 2, wobei die hohe mechanische Bettdruckfestigkeit (REL), gemessen gemäß der Methode Shell der Reihe SMS1471-74, die für Agglomerate mit einer Größe von weniger als 1,6 mm angepasst ist, größer als oder gleich wie 1,0 MPa ist, wobei die Größe dem zahlenmittleren Durchmesser des Agglomerats oder der größten zahlenmittleren Abmessung des Agglomerats entspricht, wenn das Agglomerat nicht kugelförmig ist.

4. Zeolithisches Adsorptionsmittel nach einem der Ansprüche 1 bis 3, wobei das Adsorptionsmittel eine Größe zwischen 0,1 mm und 1 mm einschließlich der Grenzwerte aufweist, wobei die Größe dem zahlenmittleren Durchmesser des Adsorptionsmittels oder der größten zahlenmittleren Abmessung des Adsorptionsmittels entspricht, wenn das Agglomerat nicht kugelförmig ist, wobei die Bestimmung der Größe durch Analyse der Korngrößenverteilung einer Probe des Agglomerats durch Bildgebung gemäß ISO 13322-2:2006 unter Verwendung eines Laufbands erfolgt, das es der Probe ermöglicht, vor dem Objektiv der Kamera vorbeizulaufen, wobei die Größe des Objekts aus der Korngrößenverteilung unter Anwendung von ISO 9276-2:2001 berechnet wird.

5. Zeolithisches Adsorptionsmittel nach einem der Ansprüche 1 bis 4, wobei der Zeolith Zeolith X ist, allein oder in Gemisch mit anderen Zeolithen.

6. Zeolith-Adsorptionsmittel nach einem der Ansprüche 1 bis 5, wobei das Adsorptionsmittel mehr als 90 Gewichts-% Zeolith(e) umfasst.

7. Zeolithisches Adsorptionsmittel nach einem der Ansprüche 1 bis 6, wobei der Zeolith oder die Zeolithe in Form von Kristallen mit einer Größe zwischen 10 nm und 1500 nm vorliegen, wobei die Größe dem zahlenmittleren Durchmesser der Kristalle oder der größten zahlenmittleren Abmessung der Kristalle entspricht, wenn die Kristalle nicht kugelförmig sind, wobei die Größe durch Beobachtung mit einem Rasterelektronenmikroskop (REM) gemessen wird.

8. Zeolithisches Adsorptionsmittel nach einem der Ansprüche 1 bis 7, wobei die Porenverteilung die folgenden Ungleichungen a) und/oder b) erfüllt:

a)

$$\frac{Vme}{Vme + Vma} \leq 0{,}1,$$

b)

$$0{,}4 \leq \frac{Vmi}{Vma + Vme + Vmi}.$$

wobei Vmi das mikroporöse Volumen in $cm^3.g^{-1}$ bezeichnet, das durch Stickstoffadsorption bestimmt wird, wobei das mikroporöse Volumen dem Volumen der Poren mit einer Öffnung von weniger als 2 nm entspricht.

9. Zeolithisches Adsorptionsmittel nach einem der Ansprüche 1 bis 8, ferner umfassend Barium und/oder Kalium.

10. Verfahren zur Herstellung eines zeolithischen Adsorptionsmittels nach einem der Ansprüche 1 bis 9, umfassend mindestens die folgenden Schritte:

a) einen Mischschritt von Kristallen mindestens eines Zeoliths mit einem Agglomerationsbindemittel, das mindestens 80 Gewichts-%, vorzugsweise mindestens 90 Gewichts-%, bevorzugter mindestens 95 Gewichts-% zeolithisierbaren Ton enthält, gefolgt von einer Formgebung und einem Brennschritt bei einer Temperatur zwischen 500 und 700 °C,
b1) einen ersten Zeolithisierungsschritt durch Inkontaktbringen des in Schritt a) erlangten Materials mit einer alkalischen basischen Lösung mit einer Konzentration zwischen 0,2 M und 0,9 M, einschließlich der Grenzwerte,
b2) einen zweiten Zeolithisierungsschritt durch Inkontaktbringen des in Schritt b1) erlangten Materials mit einer alkalischen basischen Lösung mit einer Konzentration zwischen 1,2 Met 4,0 M einschließlich der Grenzwerte, wobei b1 und b2 in beliebiger Reihenfolge durchgeführt werden können und b1 und/oder b2 wiederholt werden können,
d) einen Wasch- und Trocknungsschritt des somit erlangten Materials, und
e) einen Aktivierungsschritt des in Schritt d) erlangten Materials durch Erhitzen auf eine Temperatur zwischen 100 °C und 400 °C und Gewinnen des Zeolith-Adsorptionsmittels in Form eines Agglomerats.

11. Herstellungsverfahren nach Anspruch 10, umfassend einen Schritt c) eines kationischen Austauschs der Kationen, die in dem aus den Zeolithisierungsschritten stammenden Reaktionsmedium enthalten sind, durch Inkontaktbringen mit einer Lösung von Bariumionen oder von Bariumionen und Kaliumionen, wobei dieser Schritt zwischen Schritt b2) und d) oder zwischen Schritt b1) und d) durchgeführt wird.

12. Herstellungsverfahren nach einem der Ansprüche 10 oder 11, umfassend einen oder mehrere zusätzliche Formgebungsschritte, die nach einem der Schritte a), b1), b2), optional c), d), e) durchgeführt werden.

13. Herstellungsverfahren nach einem der Ansprüche 10 bis 12, wobei eine Siliciumdioxidquelle in dem Gemisch der Kristalle von mindestens einem Zeolithen mit dem Agglomerationsbindemittel in Schritt a) vorhanden ist.

14. Abtrennungsverfahren von para-Xylol anhand von Isomerschnitten von aromatischen Kohlenwasserstoffen, die 8 Kohlenstoffatome enthalten, in flüssiger oder gasförmiger Phase durch Adsorption des para-Xylols mittels eines Adsorptionsmittels nach einem der Ansprüche 1 bis 9 in Anwesenheit eines Desorptionsmittels.

15. Verfahren nach Anspruch 14 vom Typ simuliertes Wanderbett.

**Claims**

1. Zeolite-based adsorbent in the form of agglomerates, said adsorbent having:

   - a tortuosity factor $\tau$, of formula :

$$\tau = \left(2.23 - 1.13 \cdot V_{tot} \cdot d_g\right)\left(0.92\frac{4}{S_S}\sum_{Pi=0.21MPa}^{Pdc}\frac{\Delta Vi}{li}\right)^{1-\delta}$$

   wherein

   $V_{tot}$ represents the total pore volume, corresponding to the sum of the macropore and mesopore volumes $V_{ma}$ and $V_{me}$ expressed in cm$^3$.g$^{-1}$, $V_{tot}$, $V_{ma}$ and $V_{me}$ being measured by mercury intrusion porosimetry and related to the mass of the sample as anhydrous equivalent, $\delta$ = 0 for liquid phase or 1 for gaseous phase, $S_s$ is the surface area of the pores of macropore and mesopore type, expressed in m$^2$ per gram of sample as anhydrous equivalent, calculated according to the following equation 3 :

$$S_S = \frac{6 \cdot V_{mi}}{f \cdot d_{cristaux}} \quad \text{Formula 3}$$

   in which f is the void fraction in the crystals,
   $d_{crystals}$ is the number-mean diameter of the zeolite crystals of the zeolite-based adsorbent measured by observation with a scanning electron microscope (SEM), and $V_{mi}$ denoting the micropore volume expressed in cm$^3$.g$^{-1}$, determined by nitrogen adsorption, the micropore volume being evaluated by means of the Dubinin-Raduskevitch equation, from the isotherm obtained, by applying the standard ISO 15901-3:2007, the micropore volume corresponding to pores whose aperture is less than 2 nm,
   $\Delta Vi$ is the volume increment of mercury and li is the apparent pore diameter,
   dg is the grain density expressed in g.cm$^{-3}$ and refers to the mass of the sample as anhydrous equivalent,
   Pdc = 87 MPa,
   the tortuosity factor being calculated from the pore distribution determined by mercury intrusion porosimetry, strictly greater than 1 and strictly less than 3;
   the tortuosity factor being determined by a standard measure detailed in the user manual of Autopore® 9500 mercury porosimeter from Micromeritics ;

   - a porosity

$$\varepsilon_p = \frac{Vma + Vme}{Vg}$$

   determined by mercury intrusion porosimetry, in which $V_{ma}$ denotes the macropore volume, $V_{me}$ denotes the mesopore volume and $V_g$ denotes the grain volume, of between 25% and 35%, the volumes being expressed in cm$^3$.g$^{-1}$ ; the macropores volume corresponding to pores volume whose aperture is greater than 50 nm, the mesopores volume corresponds to pores volume whose aperture is between 2 nm and 50 nm, limits not inclusive,
   said adsorbent comprising a zeolite chosen from the zeolites of FAU structure,
   the porosimetry measurements by mercury intrusion being performed according to the standard ASTM D4284-83.

2. Zeolite-based adsorbent according to claim 1, in which the tortuosity factor $\tau$ is between 1.5 and 2.7.

3. Zeolite-based adsorbent according to claim 1 or 2, wherein the high mechanical bulk crushing strength (BCS), measured via the Shell method series SMS1471-74 adapted for agglomerates less than 1.6 mm in size, is greater than or equal to 1.0 MPa, the size means the number-mean diameter of the adsorbent or its number-mean largest dimension when it is not spherical.

4. Zeolite-based adsorbent according to any one of claims 1 to 3, wherein said adsorbent has a size of between 0.1 mm and 1 mm, limits inclusive, the size means the number-mean diameter of the adsorbent or its number-mean largest dimension when it is not spherical, the determination of the size being performed by particle size distribution analysis on a sample of agglomerate by imaging according to the standard ISO 13322-2:2006, using a conveyor belt for passing the sample before the objective lens of the camera, the size of the object being calculated from the particle size distribution by applying the standard ISO 9276-2:2001.

5. Zeolite-based adsorbent according to any one of claims 1 to 4, said zeolite being zeolite X, alone or as a mixture with other zeolites.

6. Zeolite-based adsorbent according to any one of claims 1 to 5, wherein, said adsorbent comprising more than 90% by weight of zeolite(s).

7. Zeolite-based adsorbent according to any one of claims 1 to 6, wherein said zeolite(s) are in the form of crystals between 10 nm and 1500 nm in size, the size corresponding to the number-mean diameter of the adsorbent or its number-mean largest dimension when it is not spherical, the size being measured by observation with a scanning electron microscope (SEM).

8. Zeolite-based adsorbent according to any one of claims 1 to 7, wherein the pore distribution satisfies the inequalities a) and/or b) below:

   a)

   $$\frac{Vme}{Vme + Vma} \leq 0,1,$$

   b)

   $$0,4 \leq \frac{Vmi}{Vma + Vme + Vmi}.$$

   Vmi denoting the micropore volume expressed in $cm^3.g^{-1}$, determined by nitrogen adsorption, the micropore volume corresponding to pores whose aperture is less than 2 nm.

9. Zeolite-based adsorbent according to any one of claims 1 to 8, further comprising barium and/or potassium.

10. Process for preparing a zeolite-based adsorbent according to any one of claims 1 to 9, comprising at least following steps:

    a) a step of mixing crystals of at least one zeolite with an agglomeration binder containing at least 80%, preferably at least 90%, more preferably at least 95% by weight of zeolitizable clay, followed by forming and a firing step at a temperature of between 500 and 700° C,
    b1) a first step of zeolitization by placing the material obtained in step a) in contact with an alkaline basic solution, with a concentration of between 0.2 M and 0.9 M, limits inclusive,
    b2) a second step of zeolitization by placing the material obtained in step b1) in contact with an alkaline basic solution, with a concentration of between 1.2 M and 4.0 M, limits inclusive,
    b1 and b2 possibly being performed in any order and b1 and/or b2 possibly being repeated,
    d) a step of washing and drying the material thus obtained, and
    e) a step of activation of the material obtained in step d), by heating to a temperature of between 100° C. and 400° C, and recovery of the zeolite-based adsorbent in the form of agglomerates.

11. Preparation process according to claim 10, comprising a step c) of cationic exchange of the cations contained in the reaction medium obtained from the zeolitization steps by placing in contact with a solution of barium ions or of barium ions and potassium ions, this step being performed between step b2) et d) or between steps b1) and d).

**12.** Preparation process according to claims 10 or 11, comprising one or more additional forming steps performed after any one of steps a), b1)/b2), optionally c), d) or e).

**13.** Preparation process according to any one of claims 10 to 12, wherein a source of silica is present in the mixture of crystals of at least one zeolite, with the agglomeration binder in step a).

**14.** Process for separating para-xylene from aromatic hydrocarbon isomer fractions containing 8 carbon atoms, in the liquid phase or in the gaseous phase, by absorption of para-xylene using an adsorbent according to anyone of the claims 1 to 9, in the presence of a desorbent.

**15.** Process according to claim 14, of simulated moving bed type

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- WO 2016075280 A
- FR 3004966
- WO 2009081024 A

**Littérature non-brevet citée dans la description**

- Principles of Adsorption and Adsorption Processes. Principes de l'Adsorption et des Procédés d'Adsorption. John Wiley & Sons, 1984, 326-407
- **KÄRGER** ; **RUTHVEN** ; **THEODOROU**. Diffusion in Nanoporous Materials. Wiley, 2012, vol. 1, 95
- **D.W. BRECK**. Zeolite Molecular Sieves. John Wiley and Sons, 1973, 314-315
- Construction of the tortuosity factor from porosimetry. 1986, vol. 102, 401-418
- **D.W. BRECK**. Zeolite Molecular Sieves. John Wiley and Sons, 1973